Europäisches Patentamt

(19) European Patent Office

Office européen des brevets

(11) Publication number: **0 063 953**
A2

# (12) EUROPEAN PATENT APPLICATION

(21) Application number: 82302157.1

(22) Date of filing: 27.04.82

(51) Int. Cl.³: **C 12 N 15/00**, C 12 P 21/00
// C12R1/07

(30) Priority: 29.04.81 GB 8113253

(71) Applicant: BIOGEN N.V., 15 Pietermaai, Willemstad Curacao, Netherlands Antilles (NL)

(43) Date of publication of application: 03.11.82
Bulletin 82/44

(72) Inventor: Hardy, Kimber Graham, 3 Rue Voltaire, CH-1202 Geneva (CH)
Inventor: Bingham, Allistair Harold Arthur, 12 Route des Acacias, CH-1227 Carouge (CH)

(74) Representative: Hartley, David et al, c/o Withers & Rogers 4 Dyer's Buildings Holborn, London, EC1N 2AJT (GB)

(84) Designated Contracting States: AT BE CH DE FR GB IT LI LU NL SE

(54) Bacillus cloning vectors, recombinant DNA molecules, bacillus hosts transformed with them and methods for expressing foreign DNA sequences and producing polypeptides coded thereby.

㊙ Cloning vectors, recombinant DNA molecules and Bacillus hosts transformed with them which express foreign DNA sequences and produce at least a portion of the amino acid sequences coded for by the foreign DNA sequences. The cloning vectors, recombinant DNA molecules Bacillus hosts transformed with them and methods of this invention are characterized in that they permit the cloning and expression of foreign DNA sequences, particularly those of eukaryotic and viral origin, in Bacillus hosts. In Bacillus hosts compatible with the recombinant DNA molecules of this invention, polypeptides useful in vaccines, pharmaceutical products and industrial products are produced.

EP 0 063 953 A2

BACILLUS CLONING VECTORS, RECOMBINANT
DNA MOLECULES, BACILLUS HOSTS TRANSFORMED
WITH THEM AND METHODS FOR EXPRESSING
FOREIGN DNA SEQUENCES AND PRODUCING
POLYPEPTIDES CODED THEREBY

TECHNICAL FIELD OF INVENTION

This invention relates to Bacillus cloning vectors, recombinant DNA molecules, Bacillus host transformed with them and methods for the expression of foreign DNA sequences and for producing polypeptides coded for by those sequences. More particularly, the invention relates to cloning vectors useful in Bacillus hosts for cloning and expressing foreign genes and methods for such cloning and expression. The vectors, recombinant DNA molecules, hosts and methods of this invention are characterized in that they permit the cloning and expression of foreign DNA sequences, particularly those of eukaryotic and viral origin in Bacillus hosts. As will be appreciated from the disclosure to follow, the vectors, recombinant DNA molecules, hosts and methods of this invention are useful in the production of polypeptides not usually produced in Bacillus hosts. These polypeptides are then useful in vaccines, pharmaceutical products and industrial products and as starting materials or precursors for the production of other like products by more traditional chemical processes.

BACKGROUND ART

Numerous eukaryotic and viral genes have been expressed in the gram-negative bacterium, Escherichia coli.

0063953

Examples of such expression include the production of polypeptides displaying a biological or immunological activity of human leukocyte interferon (S. Nagata et al., "Synthesis In E. coli Of A Polypeptide With Human Leukocyte Interferon Activity", Nature, 284, pp. 316-20 (1980)), antigens displaying the specificity of human hepatitis B viral antigens (C. J. Burrell et al., "Expression In Escherichia coli: Of Hepatitis B Virus Genes And Their Expression In E. coli", Nature, 282, pp. 575-79 (1979)) and polypeptides displaying the antigenicity of FMD viral antigens (H. Küpper et al., "Cloning Of cDNA Of Major Antigen Of Foot And Mouth Disease Virus And Expression In E. coli.", Nature, 289, pp. 555-59 (1981)).

However, the production of such useful eukaryotic and viral products in E. coli is characterized by several disadvantages. For example, since E. coli are routinely present in the intestinal tract of animals and humans, careful handling of E. coli hosts that have been modified to produce a desired product is required to prevent accidental infection. At present, the E. coli hosts that are permitted to be used for transformation to produce desired products are limited to those that will not grow outside of the laboratory environment. Moreover, the transformed E. coli hosts must be grown in closed systems under high conditions of containment (as now mandated by the guidelines of the National Institute of Health and other like govermental organizations). The resultant E. coli cultures must also be processed to kill the bacteria before opening the system to the environment. These requirements, obviously, greatly increase the material and equipment costs of fermentation processes using E.coli. In addition, some of the baterial products of E. coli are endotoxins. Therefore, before eukaryotic and viral products produced in E. coli hosts can be used in animals and humans, the E. coli endotoxins must be totally eliminated from the desired product. Obviously, this also entails costly and cumbersome purification and isolation procedures.

Finally, although some products made by E. coli hosts, for example, the product of the gene for pencillinase resistance, may be secreted into the periplasmic space of the E. coli cell, the products made by E. coli hosts are not usually secreted into the extracellular space of the E. coli cell. Therefore, to recover the desired products, the E. coli cells must be broken up or otherwise lysed. Such harsh treatment, of course, also frees all of the other cell products and E. coli membrane proteins from the E. coli host. Therefore, such procedures again necessitate extensive purification of the product mix in order to isolate and to recover the desired eukaryotic or viral products in a form useable in animals and humans.

These many disadvantages of E. coli hosts may in large measure be avoided by the use of Bacillus hosts to produce the desired eukaryotic and viral products. Bacillus is a gram-positive, non-pathogenic organism. It does not have the capacity to infect humans or animals. Bacillus hosts also do not produce endotoxins. Therefore, the production of eukaryotic and viral proteins in Bacillus hosts is not disadvantaged by a requirement for growth and killing under conditions of high containment or for removing all traces of toxic products before the desired products may be used in animals and humans. Bacillus hosts also do not have a periplasmic space and their cell surface is devoid of lipopolysaccharides. They also naturally secrete several of their protein products into the extracellular space. Therefore, the secreted products of Bacillus may be recovered from the extracellular space without break-up or lysis of the cell. Such procedures, of course, avoid the contamination and purification problems that attend product recovery from E.coli hosts. Finally, Bacillus hosts are well characterized and are widely used in industrial fermentations, for example, in the production of the Bacillus product $\alpha$-amylase.

However, in spite of the many advantages inherent in the use of Bacillus hosts for the expression of foreign gene products and of the many attempts to employ Bacillus hosts to effect such expression, no successful expression of eukaryotic or viral genes in Bacillus hosts has been reported.

## DISCLOSURE OF THE INVENTION

The present invention generally solves the problems referred to above by providing Bacillus cloning vectors, recombinant DNA molecules Bacillus hosts transformed with them, and methods for cloning and expressing foreign DNA sequences and producing polypeptides coded thereby in Bacillus hosts. More particularly, we provide in accordance with this invention a Bacillus cloning vehicle characterized by an intact replicon compatible with Bacillus hosts and at least one endonuclease recognition site at which such cloning vehicle may be cut in a determinable fashion without attendant loss of an essential biological function, characterized in that upon insertion of a DNA sequence, at least a portion of which sequence codes for a foreign amino acid sequence, into said endonuclease recognition site and transformation of a Bacillus host with the resultant recombinant DNA molecule, the resultant transformed Bacillus host is capable of expressing at least a portion of the DNA sequence and of producing at least a portion of the foreign amino acid sequence.

By virtue of our invention, it is for the first time possible to employ Bacillus hosts to express foreign DNA sequences, particularly those of eukaryotic and viral origin and to produce in those hosts eukaryotic and viral polypeptides that are useful in vaccine and pharmaceutical products and that have diverse industrial uses. The host-cloning vector systems, recombinant DNA molecules, and methods of our invention permit the transformation of Bacillus hosts with foreign genes that code for those polypeptides and the replication and expression of those genes by the Bacillus hosts. Thus, the host-cloning vector systems, recombinant DNA molecules and methods of this invention not only avoid the disadvantages inherent in using E. coli hosts for the replication and expression of those genes, but the cloning vectors, recombinant DNA molecules, Bacillus hosts and methods of this invention afford the advantages that characterize the fermentation, production and recovery of products in Bacillus hosts.

As will be appreciated from the disclosure to follow, the host-cloning vector systems and methods of this invention are capable of replicating and expressing genes of foreign origin to produce eukaryotic and viral polypeptides in Bacillus hosts.

### BRIEF DESCRIPTION OF THE DRAWINGS

Figure 1 is a schematic outline of one embodiment of a host-cloning vector system and a method of this invention for producing polypeptides having the specificity of hepatitis B core antigens in Bacillus hosts.

Figure 2 is a schematic outline of another embodiment of a host-cloning vector system and a method of this invention for producing polypeptides having a biological or immunological activity of human leukocyte interferon in Bacillus hosts.

Figure 3 is a schematic outline of another embodiment of a host-cloning vector system and a method of this invention for producing polypeptides having the specificity of foot and mouth disease ("FMD") viral antigens in Bacillus hosts.

### BEST MODE OF CARRYING OUT THE INVENTION

In order that the invention herein described may be more fully understood, the following detailed description is set forth.

In the description the following terms are employed:

Polypeptide-- A linear array of amino acids connected one to the other by peptide bonds between the α-amino and carboxy groups of adjacent amino acids.

Expression-- The process undergone by a DNA fragment or gene to produce a polypeptide. It is a combination of transcription (the process of producing mRNA from a DNA fragment) and translation (the process of producing a polypeptide from mRNA).

Plasmid--A non-chromosomal, double-stranded DNA sequence comprising an intact "replicon" such that the plasmid is replicated in a host cell. When the plasmid is placed within a unicellular organism, the characteristics of that organism may be changed or transformed as a result of the DNA of the plasmid. For example, a plasmid carrying the gene for tetracycline reisistance (Tet$^R$) transforms a cell previously sensitive to tetracycline into one which is resistant to it. A cell transformed by a plasmid is called a "transformant".

Phage or Bacteriophage--Bacterial virus, many of which consist of DNA sequences encapsidated in a protein envelope or coat ("capsid protein").

Bacillus Cloning Vector--A plasmid, phage DNA or other DNA sequence that has (a) an intact replicon, such that the cloning vehicle is able to replicate in a Bacillus host and (b) one or a small number of endonuclease recognition sites at which such DNA sequences may be cut in a determinable fashion without attendant loss of an essential biological function of the cloning vehicle e.g., replication, production of coat proteins or loss of promoter or binding sites. Preferably, the Bacillus cloning vector, as originally constructued or after a DNA fragment from a different genome is inserted therein, also has a marker suitable for use in the identification of transformed cells, e.g., tetracycline resistance, ampicillin resisitance or the production of a detectable polypeptide product. A Bacillus cloning vector is often called a Bacillus cloning vehicle.

Cloning--The process of obtaining a population of organisms or DNA sequences derived from one such organism or sequence by asexual reproduction.

Recombinant DNA Molecule or Hybrid DNA--A molecule consisting of segments of DNA from different genomes which have been joined end-to-end outside of living cells and have the capacity to infect some host cell and be maintained therein.

Bacillus Expression Control Sequence--A sequence of nucleotides that controls and regulates expression of structural genes in a Bacillus host when operatively linked to those genes.  They include the lac system, the trp system, major operator and promoter regions of phage $\lambda$, the control region of fd coat protein and other sequences known to control the expression of genes of prokaryotic or eukaryotic cells or their viruses.

Bacillus Host--A gram-positive, microbial strain belonging to the genus Bacillus.  It may include strains of Bacillus subtilis, Bacillus stearothermophilus, Bacillus circulans and other strains of Bacillus.

The Bacillus claiming vectors, recombinant DNA molecules, transformed hosts and methods of this invention permit the expression of foreign DNA sequences, particularly those of eukaryotic and viral origin, in Bacillus hosts and the production of the products coded for by those DNA sequences.  Examples of such products include polypeptides displaying a biological and immunological activity of leukocyte interferon, fibroblast interferon, other interferons, insulin, human and animal growth hormones, hepatitis B viral core antigens, hepatitis B viral surface antigens, FMD viral antigens and other human, animal and viral polypeptides.

In accordance with this invention, the above DNA sequences are expressed and foreign products produced by inserting a DNA sequence, at least a portion of which codes for the desired product, into a Bacillus cloning vector of this invention in such a way so as not to destroy the ability of the resultant recombinant DNA molecule to be maintained and to replicate in Bacillus hosts and so as to be operatively linked to a Bacillus expression control sequence.  The cloning vectors of this invention comprise an intact replicon compatible with Bacillus hosts and at least one endonuclease recognition site at which said cloning vector may be cut in a determinable fashion without attendant loss of an essential biological function.  They are characterized in that upon insertion of the foreign DNA sequence into the endonuclease recognition site and transformation of a <u>Bacillus</u> host with the resultant recombinant or hybrid DNA molecule, the transformed <u>Bacillus</u> host is capable of expressing at least a portion of the DNA sequence and of producing at least a portion of the amino acid sequence coded for by foreign DNA sequence.

More preferably, the <u>Bacillus</u> clong vector, either as originally constructed or after insertion of the foreign DNA sequence therein, also is characterized by a marker to enable the selection of <u>Bacillus</u> hosts transformed with a recombinant DNA molecule derived from the <u>Bacillus</u> cloning vector.

Most preferably, the <u>Bacillus</u> cloning vector of this invention is made up of a combination of at least one portion of a DNA sequence that is capable of replication in <u>E. coli</u> and at least one portion of a DNA sequence that is capable of replication in <u>Bacillus</u>. This preferred cloning vector permits transformation and selection to be done initially in <u>E. coli</u> where transformation is easier and usually of higher frequency and then the selected recombinant DNA molecule transformed to <u>Bacillus</u> hosts.

In order that this invention may be better understood, the following examples are presented by way of illustration.

### EXAMPLE I

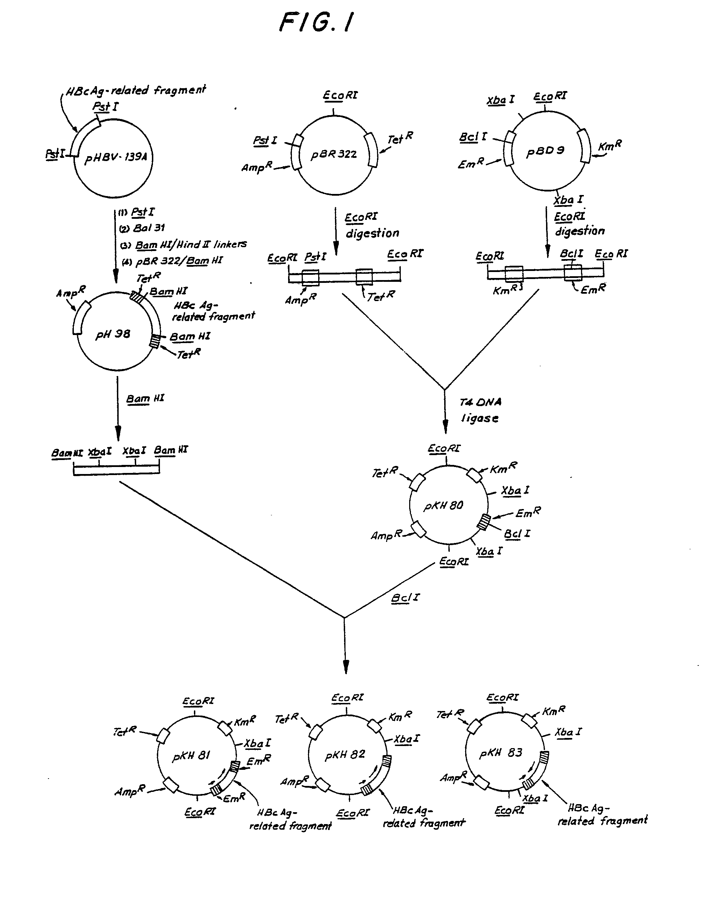

Referring now to Figure 1, we have shown therein a schematic outline of one embodiment of a <u>Bacillus</u> host-cloning vector system and a method of this invention. In this embodiment, polypeptides having the specificity of hepatitis B core antigens ("HBcAg") are produced by inserting a DNA fragment coding for that polypeptide into a <u>Bacillus</u> cloning vector of this invention and by transforming a <u>Bacillus</u> host with such vector so as to replicate and to express the inserted DNA fragment and to produce the HBcAg product.

As shown in Figure 1, a <u>Bacillus</u> cloning vector was constructed by combining <u>E. coli</u> plasmid pBR322 (F. Bolivar <u>et al.</u>, "Construction And Characterization Of New Cloning Vehicles II. A Multi-Purpose Cloning System", <u>Gene</u>, pp. 95-113 (1977); J. G. Sutcliffe, "pBR322 Restriction Map Derived From the DNA Sequence: Accurate DNA Size Markers Up To 4361 Nucelotide Pairs Long", <u>Nucleic Acids Research</u>, 5, pp. 2721-28 (1978); J. G. Sutcliffe, "Complete Nucelotide Sequence Of The <u>Eschericia coli</u> Plasmid pBR332", <u>Cold Spring Harbor Symposium</u>, 43, I. pp. 77-90 (1978)) with plasmid pBD9, a plasmid that is known to replicate in <u>Bacillus</u> hosts, "Construction And Properties Of Chimeric Plasmids In <u>Bacillus Subtilis</u>", <u>Proc Nat. Acad. Sci. USA</u>, 75, pp. 1423-28 (1978); "Characterization Of Chimeric Plasmid Cloning Vehicles In <u>Bacillus Subtilis</u>", <u>J. Bacteriol.</u> 141, pp. 246-53 (1980)).

The ligation of the two plasmids was effected by cleaving each of the plasmids with restriction exonuclease <u>Eco</u>RI and joining the two linearized plasmids in the presence of T4 DNA ligase. Since pBR322 carries the genes coding for penicillinase resistance (Amp$^R$) and tetracycline resistance (Tet$^R$) and pBD9 carries the genes coding for kanamycin resistance (Km$^R$) and erythromycin resistance (Em$^R$)* and none of these genes has been inactivated by the fusion of the two plasmids, the combined plasmid, designated pKH80 carries all four resistance markers.

Since plasmid pKH80 is a combination <u>E.coli</u> and <u>Bacillus</u> plasmid, both of those strains may be used as a host for the plasmid. Since the transformation of <u>E. coli</u> hosts is somewhat easier than the transformation of <u>Bacillus</u> hosts and particularly since the frequency of transformation is usually higher in <u>E. coli</u> hosts than in <u>Bacillus</u> hosts, it is preferable initially to transform

_____

\* Erythomycin resistance is conferred by a polypeptide of about 29,000 MW. Production of the polypeptide is induced by sub-inhibitory concentrations of erythromycin.

an E. coli host with the above-constructed plasmid and to
select correctly constructed plasmids in that host.
These plasmids may then be employed to transform the
desired Bacillus host.

Correctly constructed plasmids were selected by
transforming E. coli HB101 with the above plasmids and
selecting for resistance to ampicillin (40 µg/ml), tetra-
cycline (25 µg/ml) and kanamycin (5 µg/ml) on L-agar
plates.  The relative orientation of pBR322 and pBD9 in
pKH80 was determined by digestion with PstI, since the
two PstI sites in pKH80 are asymmetric with respect to
the pBD9 and pBR322 portions of pKH80 (Figure 1).

pKH80 also conferred erythromycin resistance on
E. coli HB101 hosts transformed with it.  Moreover, the
29,000 MW protein coded for by the gene for erythromycin
resistance was detected in E. coli minicells transformed
with pKH80 and induced with 2 µg/ml of erythromycin.

To demonstrate the utility of pKH80 as a cloning
vector in Bacillus hosts, a DNA fragment coding for a
polypeptide displaying the antigenicity of HBcAg was
selected and inserted into the cloning vector and Bacillus
hosts transformed with the recombinant or hybrid DNA
molecule to produce polypeptides displaying the antigencity of HBcAg.

As depicted in Figure 1, plasmid pHBV-139A (one
of a group of recombinant DNA molecules designated pHBV139
in C. J. Burrell et al., supra) was digested with PstI to
excise a DNA sequence containing a HBcAg-related DNA
fragment.  The excised fragment was treated under standard
conditions with exonuclease Bal31 such that an average of
100 base pairs were removed from each end of the DNA
sequence.  A mixture of BamHI and HindII linkers were
then ligated to the ends of the digested fragment.  After
BamHI digestion, the fragment was inserted into the BamHI
site of pBR322 (Figure 1).  E. coli HB101 was transformed
with these hybrid plasmids and hosts that were resistant
to ampicillin (40 µg/ml) and sensitive to tetracycline

0063953

(15 µg/ml)* were selected. Plasmid DNA was isolated from the selected transformed hosts in a conventional manner and the nucleotide sequence at the junction between pBR322 and the 5' end of the inserted HBcAg coding fragment determined by the method of A. M. Maxam and W. Gilbert, "A New Method For Sequencing DNA", Proc. Natl. Acad. Sci. USA, 74, pp. 60-64 (1977).

The nucleotide sequence of one of the isolated plasmids (pH98), together with the known sequence of the gene coding for erythromycin resistance and the location of BclI site in the gene coding for erythomycin resistance permitted the prediction that the BamHI fragment of pH98 could be inserted into the BclI site of the gene coding for erythromycin resistance in pKH80 in a proper reading frame such that the termination codon that precedes by three nucleotides the AUG start codon of the HBcAg related DNA fragment in the BamHI fragment from pH98 would be in phase with the gene for erythromycin resistance in pKH80. Therefore, it was expected that in these constructions expression of the erythromycin gene would begin at its usual start codon and would be terminated at the stop codon provided by the HBcAg insert. De novo expression would then begin again at the AUG start codon of the HBcAg-related DNA fragment to produce an unfused product displaying the antigencity of HBcAg.

Accordingly, the BamHI fragment of pH98 was inserted into the BclI site of pKH80.** As before,

---

* The BamHI site is located in the gene coding for tetracycline resistance in pBR322. Therefore, the insertion of a DNA fragment at that site inactivates the gene coding for tetracycline resistance and renders a host transformed with the resultant recombinant DNA molecule sensitive to tetracycline.

** The DNA fragments produced by digestion with BamHI and BclI have the sequence GATC at their 5' ends so that a fragment produced by digestion with BamHI can be inserted into a BclI site.

correctly constructed recombinant DNA molecules were selected after transformation of E. coli HB101 (now by resistance to ampicillin (40 μg/ml) and tetracycline (25 μg/ml) and sensitivity to erythromycin (60 μg/ml)). This combination of antibiotic sensitivity and resistance demonstrates that the E. coli host has been transformed with a plasmid conveying resistance to tetracycline and ampicillin and not conveying resistance to erythromycin (i.e., a hybrid molecule derived from pKH80, but with the gene coding for erythromycin resistance inactivated by insertion of a foreign DNA fragment into it).

The selected recombinant DNA molecules were isolated from the E. coli hosts by conventional methods of isolating plasmid DNA and the hybrid molecules digested with XbaI to determine the orientation of the inserted fragments. Of the three plasmids selected, two, pKH81 and pKH82, had the DNA fragment coding for HBcAg in the same orientation as the gene coding for erythromycin resistance. The other isolated plasmid pKH83 had the HBcAg-related DNA fragment inserted in the opposite direction (Figure 1).

Bacillus subtilis strains MI119 (leu B6, trpC2, r⁻, m⁻) ("Restriction Of Plasmid Mediated Transformations In Bacillus subtilis 168", Mol. Gen. Genet., 175, pp. 235-37 (1979)) and SL650 (spoIIA69, trpC2, rif-2) (a gift of R. Piggot)* were transformed with pKH81, pKH82 and pKH83 using conventional methods (Contente and Dubnau, "Characterization Of Plasmid Transformations In Bacillus Subtilis: Kinetic Properties And The Effect Of DNA Conformation, Mol. Gen. Genet., 167, pp. 251-58 (1979)). Appropriately transformed Bacillus hosts were then selected by resistance to kanamycin and sensitivity to erythromycin. The structure of the plasmids in these selected transformed Bacillus hosts was also confirmed by isolating the plasmid DNA

---

*    Strain SL650 does not produce the spores or proteases that are commonly produced by sporulation-proficient B. subtilis.

from the host (Lovett and Keggins, "Bacillus subtilis As A Host For Molecular Cloning", in Methods in Enzymol., 68, pp. 342-47 (1979)), and digesting the plasmid DNA with XbaI.

The Bacillus hosts that had been transformed with pKH81, pKH82 and pKH83 were assayed after induction with sub-inhibitory concentration (0.05 mg/ml) of erythromycin (in order to induce synthesis of polypeptides inserted in the gene coding for erythromycin resistance) for the production of products displaying the antigenicity of HBcAg by solid phase radioimmunoassay (see e.g., C. J. Burrell et al., supra). The results were as follows:

| Transformed Strain | Radio immunoassay (HBcAg) |
|---|---|
| B. subtilis MI119 (pKH81) | + |
| B. subtilis MI119 (pKH82) | + |
| B. subtilis MI119 (pKH83) | - |
| B. subtilis SL650 (pKH81) | + |
| B. subtilis SL650 (pKH82) | + |
| B. subtilis SL650 (pKH83) | - |

EXAMPLE II

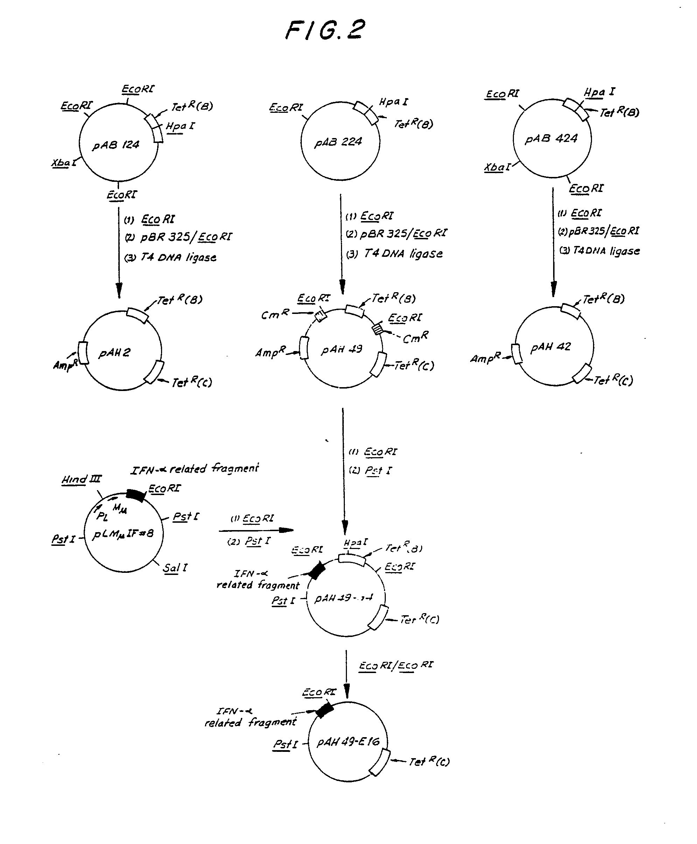

Referring now to Figure 2, we have shown therein a schematic outline of another embodiment of a Bacillus host-cloning vector system and method of this invention. In this embodiment polypeptides having a biological or immunological activity of human leukocyte interferon ("IFN-α") are produced by inserting a DNA fragment coding for that polypeptide into a Bacillus cloning vector of this invention and transforming a Bacillus host with such vectors to enable the inserted DNA fragment to be replicated and to be expressed in the Bacillus host and to produce the IFN-α product.

As shown in Figure 2, another Bacillus cloning vector of this invention was constructed by combining plasmid pAB124, isolated from a thermophilic Bacillus strain, and its deletion derivatives pAB224 and pAB424 (A.H.A. Bingham et al., Jour. Gen. Microbiol., 114, pp. 401-08 (1979) A.H.A. Bingham et al., Jour. Gen. Microbiol., 119, pp. 109-115 (1980)) with plasmid pBR325. The ligation of the two plasmids was effected by cleaving each of the plasmids with EcoRI and joining the two linearized plasmids in the presence of T4 DNA ligase. Since pBR325 carries the E. coli genes coding for penicillinase resistance (Amp$^R$) and tetracycline resistance (Tet$^R$) and pAB124, pAB224 and pAB424 carry the Bacillus gene coding for tetracyline resistance (Tet$^R$) and none of these genes has been inactivated by the fusion, the combined plasmids, designated pAH2 (from pAB124), pAH49 (from pAB224) and pAH42 (from pAB424) carry an ampicillin resistance marker and both the E. coli and Bacillus tetracycline resistance markers. Correctly constructed plasmids were selected by transforming E. coli HB101 with the plasmids and selecting for resistance to ampicillin and tetracycline and sensitivity to chloramphenicol.*

The chimeric plasmids or cloning vectors produced above were stable in E. coli strains HB101, DS410 and DB11 and in Bacillus strains BD224 and MI119. No deletions or rearrangements were observed in plasmids re-isolated from these strains after initial transformation.

To demonstrate the utility of pAH49 as a cloning vector in Bacillus hosts, a DNA fragment coding for polypeptides displaying a biological or immunological activity of IFN-α was selected and inserted into the cloning vector and Bacillus hosts transformed with the recombinant or hybrid DNA molecule to produce polypeptides displaying a biological or immunological activity of IFN-α.

Plasmid pLMμIF-8, a plasmid containing a DNA fragment coding for IFN-α(1) or 2h (S. Nagata et al., supra) promoted by pLMμ (Figure 2) was digested with

---

* The correctly constructed plasmids are sensitive to chloramphenicol ("Cm$^R$") because pBR325 includes the gene coding for chloramphenicol resistance but that gene has been inactivated by the insertion into its E. coli site of the Bacillus plasmids.

EcoRI and PstI to remove the 1.8 Kb EcoRI-PstI fragment that contains the IFN-α gene. This fragment was then employed to replace the 0.98 Kb EcoRI-PstI fragment of pAH49. The resulting hybrid or recombinant DNA molecule was selected by sizing and was designated pAH49-G4.

E. coli HB101 was transformed with pAH49-G4 and a deletion derivative of that plasmid pAH49-E16.* In addition, Bacillus subtilis SL650 and MI119 hosts were transformed with pAH49-G4. Interferon production by these transformed hosts was assayed in a conventional manner (S. Nagata et al., supra.) The results were as follows:

| Transformed Strain | Units IFN/liter |
| --- | --- |
| E. coli. HB101 (pAH49-G4) | $100\text{-}200 \times 10^6$ |
| E. coli. HB101 (pAH49-E16) | $5\text{-}10 \times 10^6$ |
| B. subtilis MI119 (pAH49-G4) | $1\text{-}5 \times 10^6$ |
| B. subtilis SL650 (pAH49-G4) | $1\text{-}2 \times 10^6$ |

## EXAMPLE III

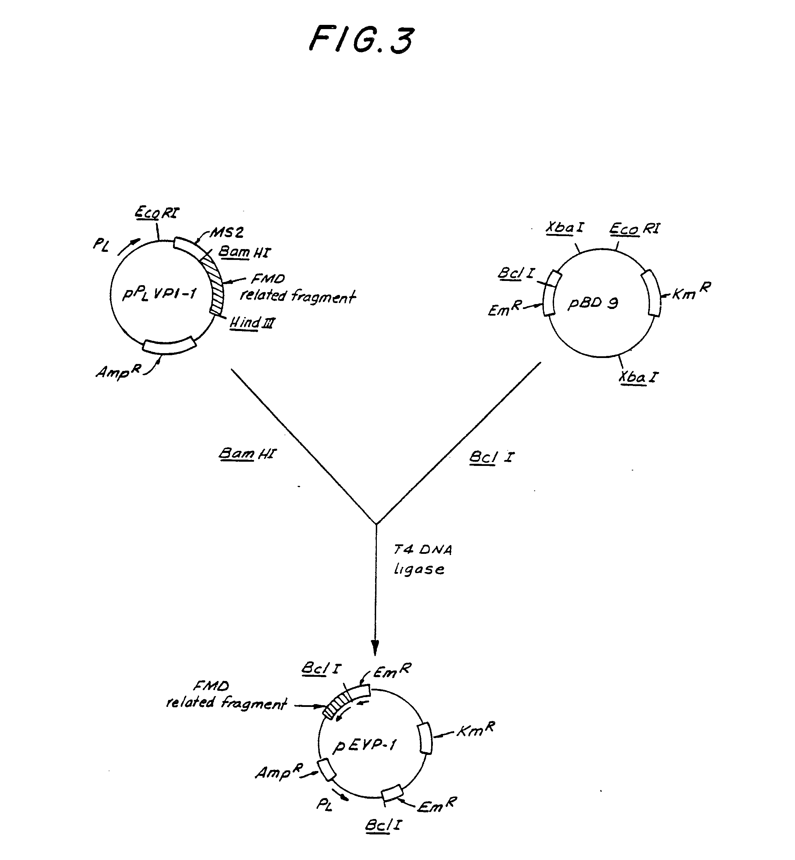

Referring now to Figure 3, we have shown therein a schematic outline of another embodiment of a Bacillus host-cloning vector system and method of this invention. In this embodiment polypeptides displaying the specificity of FMD viral antigens are produced by inserting a DNA fragment coding for that polypeptide into a Bacillus cloning vector and transforming a Bacillus host with such vector to enable the inserted DNA fragment to be replicated and to be expressed in a Bacillus host and to produce the antigenic product.

As shown in Figure 3, plasmid pBD9 (supra) was linearized with BclI and the linearized plasmid joined to BamHI-linearized plasmid pP_LVP1-1 (Küpper et al., supra) in the presence of T4 DNA ligase. Bacillus subtilis BD170 (Contente and Dubnau, supra) was then transformed

---

* Plasmid pAH49-E16 resulted from the deletion of the EcoRI-EcoRI fragment containing the Bacillus tetracycline resistance gene (Figure 2).

with the resultant recombinant DNA molecule and appropriately constructed plasmids selected by sensitivity of the transformed hosts to erythromycin (5mg/ml). These were designated pEVP-1.

None of the above-described polypeptides was observed to have been secreted extracellularly of the Bacillus host transformed with the plasmids described in Examples I-III. However, such behavior was expected, because none of the DNA sequences that coded for such products included or were fused to a DNA signal sequence that would be recognized and correctly processed for secretion by Bacillus hosts. Signal sequences that are recognized by Bacillus hosts are known. They include, for example, this signal sequence for the penicillinase gene of Bacillus lichenoformis (determined by H. Schaller, private communication) and the signal sequence for the gene coding for $\alpha$-amylase. Therefore, construction of a plasmid to provide a foreign gene with such signal sequence will permit secretion of the foreign gene's product from the Bacillus host. Such constructions do not depart from the scope of the invention and should be considered as an integral part thereof.

Upon induction with erythromycin (0.05 mg/ml), polypeptides were produced by the selected transformed Bacillus hosts that displayed the specificity of FMD viral antigens in assays essentially as described by Küpper et al., supra.

Microorganisms and recombinant DNA molecules prepared by the processes described herein are exemplified by cultures deposited in the culture collection of Deutsche Sammlung Van Mikroorganism in Gottingen, West Germay, on April 27, 1981 and identified as A to C:

A: Bacillus subtilis MI119 (pKH81)
B: Bacillus subtilis BD170 (pEVP-1)
C: Bacillus subtilis MI119 (pAH49-G4)

These cultures have been assigned accession numbers DSM 2084 which identifies bacterial strain MI119 (pKH81), DSM 2085 which identifies bacterial strain BD170 (pEVP-1) and DSM 2086 which identifies bacterial strain MI119 (pAH49-G4).

While we have hereinbefore presented a number of embodiments of this invention, it is apparent that our basic construction can be altered to provide other embodiments which utilized the processes and compositions of this invention. Therefore, it will be appreciated that the scope of this invention is to be defined by the claims appended hereto rather than the specific embodiments which have been presented hereinbefore by way of example.

Claims

1.   A <u>Bacillus</u> cloning vector comprising an intact replicon compatible with <u>Bacillus</u> hosts and at least one endonuclease recognition site at which said cloning vector may be cut in a determinable fashion without attendant loss of an essential biological function, characterized in that upon insertion of a DNA sequence at least a portion of which sequences codes for a foreign amino acid sequence into said endonuclease recognition site and transformation of a <u>Bacillus</u> host with the resultant recombinant DNA molecule, the transformed <u>Bacillus</u> host is capable of expressing at least a portion of said DNA sequence and of producing at least a portion of said foreign amino acid sequence.

2.   The <u>Bacillus</u> cloning vector according to claim 1, also being characterized either as originally constructed or after insertion therein of said DNA sequence by at least one marker suitable for use in identifying <u>Bacillus</u> hosts that have been transformed with said recombinant DNA molecule.

3.   The <u>Bacillus</u> cloning vector according to claim 1 or 2, characterized in that said cloning vehicle is a combination of at least one portion of a DNA sequence that is capable of replication in <u>E. coli.</u> and at least one portion of a DNA sequence that is capable of replication in <u>Bacillus</u>.

4.   The <u>Bacillus</u> cloning vector according to any one of claims 1 to 3, characterized in that it is selected from the group consisting of pKH80, pAH2, pAH49, pAH28 pEVP-1 and derivatives thereof.

5.   A method for constructing a <u>Bacillus</u> cloning vector according to claim 3 or 4, characterized by the step of combining at least one portion of a DNA sequence that is capable of replication in <u>E. coli</u> with at least one portion of a DNA sequence that is capable of replication in <u>Bacillus</u>, said step of combining the two DNA sequences not being destructive of the ability of the cloning vector to replicate in Bacillus.

6. A recombinant DNA molecule characterized by a Bacillus cloning vector according to any one of claims 1 to 4 and a DNA sequence, at least a portion of which sequence codes for a eukaryotic or viral amino acid sequence, said DNA sequence being inserted into said Bacillus cloning vector so as not to destroy the ability of said recombinant DNA molecule to replicate in Bacillus hosts and so as to be operatively linked to a Bacillus expression control sequence.

7. The recombinant DNA molecule according to claim 6, characterized in that said eukaryotic or viral amino acid sequence displays an immunological or biological activity of polypeptides selected from the group consisting of leukocyte interferon, fibroblast interferon, other interferons, insulin, human and animal growth hormones, hepatitis B viral core antigens, hepatitis B viral surface antigens, FMD viral antigens and other human, animal and viral polypeptides.

8. A method for constructing a recombinant DNA molecule according to claim 6 or 7, characterized by the step of inserting a DNA sequence, at least a portion of which codes for a eukaryotic or viral amino acid sequence, into a Bacillus cloning vector according to any one of claims 1 to 4, said step of inserting said DNA sequence not being destructive of the ability of said recombinant DNA molecule to replicate in Bacillus.

9. A method of producing a polypeptide at least a portion of which is of eukaryotic or viral origin in a Bacillus host, characterized by the steps of culturing a Bacillus host transformed with a recombinant DNA molecule according to claim 6 or 7 and collecting said polypeptide.

10. The method of claim 9, characterized in that said polypeptide displays an immunological or biological activity of polypeptides selected from the group consisting of leukocyte interferon, fibroblast interferon, other interferons, insulin, human and animal growth hormones, hepatitis B viral core antigens, hepatitis B viral surface antigens, FMA viral antigens and other human, animal and viral polypeptides.

*FIG. 1*

FIG. 2

# FIG.3